# EUROPEAN PATENT APPLICATION

(11) **EP 4 764 455 A1**
(43) Date of publication of application: **24.06.2026**
(21) Application number: 24947713.4
(22) Date of filing: 01.11.2024
(51) Int. Cl.: G01N 21/359, G01N 21/3563, G01N 21/76

(54) **NEAR-INFRARED-II CHEMILUMINESCENCE RESONANCE ENERGY TRANSFER NANOSYSTEM, PREPARATION METHOD THEREFOR, AND USE THEREOF**

(30) Priority: 16.07.2024 CN 202410948618
(71) Applicant: Suzhou Institute of Nano-Tech and Nano-Bionics (SINANO), Chinese Academy of Sciences, Suzhou, Jiangsu 215123 (CN)
(72) Inventor: WANG, Qiangbin, Suzhou, Jiangsu 215123 (CN); LING, Sisi, Suzhou, Jiangsu 215123 (CN); LI, Chunyan, Suzhou, Jiangsu 215123 (CN); YANG, Hongchao, Suzhou, Jiangsu 215123 (CN); ZHANG, Yejun, Suzhou, Jiangsu 215123 (CN)
(74) Representative: Bayramoglu et al.
(86) International application number: PCT/CN2024/129378
(87) International publication number: WO 2026/016334

(57) **Abstract**

A Near-infrared II (NIR-II, 1000-1700 nm) chemiluminescence resonance energy transfer (CRET) nanosystem, and a preparation method therefor and application thereof are provided. The NIR-II CRET nanosystem is constructed through multicomponent coordinated self-assembly of a pH-responsive amphiphilic short peptide, a metal ion, an NIR-II CRET functional unit, and a photosensitizer. The NIR-II CRET nanosystem features pH and MPO dual-responsive activation of NIR-II CRET signals, which can be used as a diagnostic reagent to achieve accurate tracing and localization of sentinel lymph nodes (SLNs) in breast cancer through the near-infrared II (NIR-II) contrast agent and achieve identification of SLNs through the pH and MPO dual-responsive activated NIR-II CRET signal, thus overcoming the technical problems of false-negative and false-positive which are difficult to be avoided by the traditional diagnostic methods of SLNs, and realizing the integrated localization and identification for the diagnosis and treatment of metastatic SLNs in breast cancer at an *in vivo* level.

## Description

The present application claims the priority of Chinese Patent Application No. CN 202410948618.4, entitled with "NEAR-INFRARED II CHEMILUMINESCENCE RESONANCE ENERGY TRANSFER NANOSYSTEM, PREPARATION METHOD THEREFOR AND APPLICATION THEREOF", filed on July 16, 2024, which is incorporated by reference herein in its entirety.

### TECHNICAL FIELD

The present disclosure relates to the field of optical inspection technology, particularly to a Near-infrared II (NIR-II) chemiluminescence resonance energy transfer (CRET) nanosystem and a preparation method therefor and application thereof.

### BACKGROUND TECHNOLOGY

Breast cancer is one of the most significant public health issues endangering women's health. Metastasis of breast cancer is a leading cause of death among clinical patients. Therefore, early detection and effective intervention of breast cancer are essential for improving the treatment outcomes and enhancing the quality of life for patients. Clinical studies have shown that breast cancer cells tend to spread preferentially through lymphatic metastasis. Among them, sentinel lymph nodes (SLNs) are considered as the first site where breast cancer cells arrive after spreading. As such, accurate diagnosis of metastatic SLNs is critical for clinical classification and staging of breast cancer, which is of great clinical significance in guiding the subsequent treatment.

Sentinel lymph node biopsy for breast cancer is currently the most common method for the diagnosis of SLNs, which involves obtaining tissue samples first form a solid tumor through surgical or puncture procedures, followed by *ex vivo* processing and pathological staining evaluation. Although the above method has benefited many patients, there are still problems remain: first, it is difficult to obtain complete information on sentinel lymph node metastasis through partial tissue extraction; second, invasive tissue extraction increases the risk of metastasis; in addition, identification of the results requires a relatively long process of pathologic evaluation. Therefore, how to achieve rapid and accurate diagnosis of SLNs in breast cancer remains an urgent clinical need to be addressed.

Fluorescence molecular imaging technology possesses a series of advantages such as high sensitivity, high specificity, intuitive visualization, and operational convenience. Among them, Near-infrared II (NIR-II, 1000-1700 nm) fluorescence imaging technology demonstrates excellent tissue penetration ability, spatial resolution and low tissue autofluorescence, which significantly improves the accuracy and sensitivity of cancer diagnosis. The pathological characteristics of the tumor microenvironment have been applied to develop a variety of activated nano-fluorescent probes within tumor tissues. However, it is difficult to ensure accurate results with a single detection indicator. A synergistic diagnostic strategy based on multiple indicators is an effective way to improve the accuracy.

Studies have shown that subacidity is an essential hallmark of solid tumors, including breast cancer, regardless of the stage of tumorigenesis and progression. In addition, tumor-associated macrophages (TAMs) and neutrophils (TANs) constitute a significant proportion of metastatic breast cancer; among them, myeloperoxidase (MPO) is a critical component of both TAMs and TANs, which is deeply involved in tumor metastasis. Therefore, specific identification of signals from MPO-assay can emerge as a prominent target for detecting metastatic SLNs.

In view of the limitations of the current SLN metastasis identification techniques, this disclosure provides a method of NIR-II CRET technology based on dual-responsive activation triggered by pH and MPO, and the method has been successfully incorporated into a pharmaceutical formulation enabling integrated localization and identification for the diagnosis and treatment of SLNs in breast cancer.

### CONTENT OF THE INVENTION

The present disclosure provides a technical method, namely, a near-infrared II chemiluminescence resonance energy transfer (NIR-II CRET) nanosystem and a preparation method therefor and application thereof, which is intended to achieve rapid and accurate diagnosis and treatment of SLNs in breast cancer.

In order to achieve above-mentioned objectives, the present disclosure provides a NIR-II CRET nanosystem. This nanosystem is constructed through multicomponent coordinated self-assembly of a pH-responsive amphiphilic short peptide, a metal ion, an NIR-II CRET functional unit, and a photosensitizer. The NIR-II CRET nanosystem features pH and MPO dual-responsive activation of NIR-II CRET signals.

Preferably, the pH-responsive amphiphilic short peptide is any one of and any combination of two or more of N-fluorenylmethyloxycarbonyl-L-histidine (Fmoc-His), N-fluorenylmethyloxycarbonyl-L-lysine (Fmoc-Lys), N-fluorenylmethyloxycarbonyl-L-arginine (Fmoc-Arg), and N-fluorenylmethyloxycarbonyl-L-cysteine (Fmoc-Cys).

Preferably, the metal ion is one of or any combination of two or more of gadolinium ion (Gd³⁺), erbium ion (Er³⁺), ferric ion (Fe³⁺), calcium ion (Ca²⁺), copper ion (Cu²⁺), magnesium ion (Mg²⁺), zinc ion (Zn²⁺), and manganese ion (Mn²⁺).

Preferably, the NIR-II CRET functional unit includes an NIR-II contrast agent and a chemiluminescent compound.

More preferably, the chemiluminescent compound is luminol or a luminol derivative; and the luminol derivative is selected from 4-aminohexyl-N-ethylisoluminol (ABEI) and/or ethyl luminol (AHEI).

Preferably, the NIR-II contrast agent includes at least one of the contrast agents including NIR quantum dots (QDs).

Preferably, the NIR QDs are modified by one of or any combination of two or more of mercaptopropionic acid, undecanoic acid, glutathione, and lipoic acid.

More preferably, the NIR QDs are one of or any combination of two or more of Ag₂S, Ag₂Se, AgTe, Au:Ag₂Te, and AgAuSe.

Preferably, the photosensitizer is one of or any combination of two or more of chlorin e6 (Ce6), benzoporphyrin derivative (BPD), 2-(1-hexaoxyethyl)-2-devinyl pyrophosphate (HPPH), and benzoporphyrin monoacid ring A (BPD-MA).

In another aspect, the present disclosure further provides a preparation method for the NIR-II CRET nanosystem. The method includes: blending a pH-responsive amphiphilic short peptide, a metal ion, an NIR-II CRET functional unit, and a photosensitizer, followed by multicomponent coordinated self-assembly to fabricate the NIR-II CRET nanosystem. The multicomponent coordinated self-assembly mechanism involves synergistic interactions including electrostatic adsorption, metal coordination, hydrophobic interactions, and π-π stacking among the pH-responsive amphiphilic short peptide, the metal ion, the NIR-II CRET functional unit, and the photosensitizer.

Specifically, the preparation method includes the following steps:
(1) providing a solution of NIR-II CRET functional units;
   an NIR-II contrast agent and a chemiluminescent compound are respectively dissolved in water to obtain a solution of the contrast agent and a solution of the chemiluminescent compound, which are mixed and continuously stirred for 30-120 minutes, and then purified to obtain a preparation method for the NIR-II CRET functional units;
(2) providing an amphiphilic short peptide solution, a metal ion solution and a photosensitizer solution;
   a pH-responsive amphiphilic short peptide is dissolved in water to obtain the amphiphilic short peptide solution;
   a metal ion is dissolved in water to obtain the metal ion solution;
   a photosensitizer is dissolved in an organic solvent to obtain the photosensitizer solution;
(3) preparing the NIR-II CRET nanosystem;
   the purified solution containing NIR-II CRET functional units obtained from step (1) is mixed with the amphiphilic short peptide solution and the metal ion solution from step (2), followed by continuously stirring for 10-40 minutes; the photosensitizer solution prepared in step (2) is then added and mixed uniformly, adjusted to neutral pH and purified, and concentrated to obtain the NIR-II CRET nanosystem.

Preferably, in step (1) and step (4), the purification steps were all performed by ultrafiltration.

Preferably, the preparation method for the chemiluminescent compound solution includes dissolving the chemiluminescent compound in an alkaline solution; and the pH of alkaline solution is about 8.0-9.0.

Preferably, a mass ratio of the NIR-II contrast agent to the chemiluminescent compound is in a range of 1-10:0.1-1.

Preferably, in step (2), the organic solvent is dimethyl sulfoxide.

Preferably, the concentration of amphiphilic short peptide solution is 1-5 mg/mL.

Preferably, the concentration of metal ion solution is 5-20 mmol/mL.

Preferably, the concentration of photosensitizer solution is 1-10 mg/mL.

Preferably, in step (3), a mass ratio of the NIR-II contrast agent, the chemiluminescent compound, the amphiphilic short peptide, the metal ion and the photosensitizer contained in the NIR-II CRET nanosystem is in a range of 1-10:50-100:5-50:50-200:1-5.

The pH value of the NIR-II CRET nanosystem is neutral; the average size of NIR-II CRET nanosystem under neutral conditions is in a range of 20-500 nm.

Based on the aforementioned technical solutions, this disclosure constructs an NIR-II CRET nanosystem through the integration of a pH-responsive amphiphilic short peptide, a metal ion, an NIR-II CRET functional unit and a photosensitizer via multicomponent coordinated self-assembly. The mechanism of multicomponent coordinated self-assembly involves electrostatic adhesion, metal coordination, hydrophobic interaction, π-π stacking among the pH-responsive amphiphilic short peptide, the metal ion, the NIR-II CRET system and the photosensitizer. As a novel diagnostic platform, this NIR-II CRET nanosystem establishes an integrated universal protocol combining accurate localization with molecular identification for SLN management in breast cancer, thereby offering a novel clinical approach to SLN diagnosis and therapy through NIR-II CRET nanotechnology.

The present disclosure has achieved the following beneficial technical effects:
(1) The present disclosure integrates a dual-mode optical and pH/MPO synergistically activated strategy to achieve accurate localization and diagnosis of metastatic SLNs, thus effectively minimizing both false-negative and false-positive outcomes.
(2) Benefiting from the high tissue penetration, high temporal-spatial resolution and high signal-to-noise ratio imaging properties in the NIR-II window, the use of the technical solution of the present disclosure facilitates the rapid detection of deeper and earlier metastatic lesions.

### DESCRIPTION OF THE DRAWINGS

FIG. 1 shows the transmission electron microscopy characterization images of the pH and MPO dual-responsive activated NIR-II CRET nanosystem prepared in Example 1 of the present disclosure under different pH environments.
FIG. 2 shows the transmission electron microscopy characterization images of the pH and MPO dual-responsive activated NIR-II CRET nanosystem prepared in Example 2 of the present disclosure under different pH environments.
FIG. 3a and FIG. 3b show the CRET spectra and fluorescence quantitative curves generated by the NIR-II CRET nanosystem of Example 2 from the present disclosure *in vitro* by the MPO catalyzed reaction, respectively.
FIG. 4 shows the effect of the pH and MPO dual-responsive activated NIR-II CRET nanosystem prepared in Example 1 of the present disclosure for the diagnosis of metastatic SLNs in breast cancer at an *in vivo* level.
FIG. 5 shows the effect of immunofluorescence staining and hematoxylin-eosin staining (H&E) of metastatic SLN tissues and negative SLN tissues removed under the guidance of CRET signals.

### SPECIFIC IMPLEMENTATIONS

In order to make the purpose, technical solutions and advantages of the embodiments of the present application clearer, the technical solutions in the embodiments of the present application will be described clearly and completely below in conjunction with the accompanying drawings in the embodiments of the present application, and it is obvious that the described embodiments are a part of the embodiments of the present application, rather than all the embodiments.

The present disclosure provides a pH and MPO dual-responsive activated NIR-II CRET nanosystem, which includes a pH-responsive amphiphilic short peptide, a metal ion, an NIR-II CRET functional unit, and a photosensitizer. The nanosystem is prepared by the coordinated self-assembly of the above multiple components through electrostatic adhesion, metal coordination, hydrophobic interaction, π-π stacking and other interactions.

The nanosystem enables precise localization of SLNs via NIR-II fluorescence signals, and the identification of SLNs through a dual-responsive mechanism triggered by pH and MPO in the microenvironment of breast cancer tumor, which overcomes the difficulty of integrated localization and identification for the diagnosis of SLNs *in situ* by traditional methods, and provides a new approach for the accurate diagnosis and treatment of metastatic SLNs in breast cancer.

In some specific embodiments, the pH-responsive amphiphilic short peptide is any one of and any combination of N-fluorenylmethyloxycarbonyl-L-histidine (Fmoc-His), N-fluorenylmethyloxycarbonyl-L-lysine (Fmoc-Lys), N-fluorenylmethyloxycarbonyl-L-arginine (Fmoc-Arg), and N-fluorenylmethyloxycarbonyl-L-cysteine (Fmoc-Cys).

In some specific embodiments, the metal ion is one of or any combination of gadolinium ion (Gd³⁺), erbium ion (Er³⁺), ferric ion (Fe³⁺), and zinc ion (Zn²⁺),

In some specific embodiments, the NIR-II CRET functional unit is loaded with any combination containing a NIR-II contrast agent and a chemiluminescent compound.

In some specific embodiments, the photosensitizer is one of or any combination of chlorin e6 (Ce6), benzoporphyrin derivative (BPD), 2-(1-hexaoxyethyl)-2-devinyl pyrophosphate (HPPH), benzoporphyrin monoacid ring A (BPD-MA).

In some specific embodiments, the nanosystem includes at least one of the contrast agents including NIR QDs.

In some specific embodiments, the NIR QDs are one of or any combination of Ag₂S, Ag₂Se, AgTe, Au:Ag₂Te, and AgAuSe.

In some specific embodiments, the NIR QDs are modified by one of or any combination of mercaptopropionic acid, undecanoic acid, glutathione, and lipoic acid.

In some specific embodiments, the chemiluminescent compound is one of or any combination of luminol or derivatives thereof, 4-aminohexyl-N-ethylisoluminol (ABEI) and ethylluminol (AHEI).

In another aspect, the present disclosure provides a preparation method for the pH and MPO dual-responsive activated NIR-II CRET nanosystem, which includes the following steps:
(1) dissolving the NIR-II contrast agent and the chemiluminescent compound in water, respectively, to obtain two solutions;
(2) mixing the two solutions obtained in (1) and continuously stirring for 1 h, then purifying to remove excessive chemiluminescent compound;
(3) dissolving the amphiphilic short peptide and the metal ion in water, respectively, and dissolving the photosensitizer in an organic solvent to obtain a photosensitizer solution through ultrasonication;
(4) mixing the solution of NIR-II CRET functional units obtained after purification in (2) and the amphiphilic short peptide solution obtained in (3) simultaneously with the metal ion solution obtained in (3) and continuously stirring for 30 minutes, then adding the photosensitizer solution obtained in (3), and adjusting the pH of the mixed solution to neutral, followed by purification and concentration, to obtain the solution of the pH and MPO dual-responsive activated nanosystem.

The present disclosure provides the pH and MPO dual-responsive activated NIR-II CRET nanosystem, which enables integrated localization and identification of SLNs in breast cancer, thereby improving diagnostic and therapeutic outcomes.

It should be noted that, unless otherwise specified, the raw materials and chemical reagents selected for the present disclosure are all commercially available.

The technical solutions in the embodiments of the present disclosure are hereinafter described clearly and completely by means of specific embodiments and the accompanying drawings, and it is obvious that the described embodiments are only a part of the embodiments of the present disclosure, rather than all the embodiments. Based on the embodiments in the present disclosure, all other embodiments obtained by a person of ordinary skill in the art without making any creative work fall within the scope of protection of the present disclosure.

### Example 1

This example provides a preparation method for a pH and MPO dual-responsive activated NIR-II CRET nanosystem, which specifically includes the following steps:
(1) 10 mg of 4-(N-ethyl-N-amino butylamino) phthalylhydrazine (ABEI), 0.5 g of sodium bicarbonate and 200 µL of strong aqua ammonia were dissolved in water to obtain an ABEI solution;
(2) 1 mg of undecanoic acid-modified Ag₂S QDs were dissolved in water to obtain an aqueous solution of QDs (at an average concentration of 0.5 mg/mL);
(3) the ABEI solution obtained in (1) and the quantum dot solution obtained in (2) were mixed at a mass ratio of 100:1 and continuously stirred for 1 h at a stirring speed of 300 rpm;
(4) the solution obtained in (3) was purified three times by ultrafiltration, for 10 minutes each time, to remove excessive ABEI to obtain a purified solution containing NIR-II CRET functional units;
(5) 1 mg of fluorenylmethyloxycarbonyllysine (Fmoc-Lys) and 5 mmol of ErCl₃ were respectively dissolved in 1 mL of water to obtain an Fmoc-Lys solution and an ErCl₃ solution, respectively; and 1 mg of dichlorophenyl phosphine (BPD) was dissolved in 1 mL of DMSO to obtain a BPD solution;
(6) the solution of NIR-II CRET functional units obtained in step (4) and the Fmoc-Lys solution obtained in (5) were simultaneously mixed with the ErCl₃ solution and continuously stirred for 30 minutes at a stirring speed of 300 rpm to finally obtain a mixed solution;
(7) the BPD solution formulated in step (5) and the mixed solution formulated in step (6) were mixed uniformly and then divided into two portions, which were respectively adjusted to pH = 7.4 and pH = 5.5, and continuously stirred for 1 h, and then concentrated by ultrafiltration to obtain a purified solution of the NIR-II CRET nanosystem.

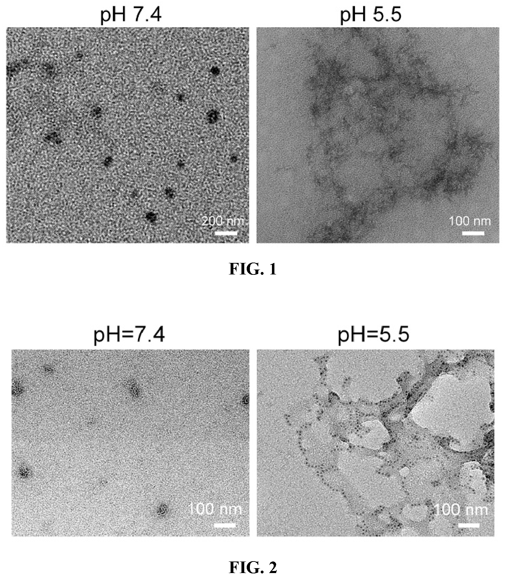

As shown in FIG. 1, it shows the morphology characterization images of the NIR-II CRET nanosystem provided in this example. It can be seen from the transmission electron microscopy images that the nanosystem has a size of about 120 nm at pH 7.4 and about 30 nm at pH 5.5; indicating that the NIR-II CRET nanosystem is successfully prepared in an environment of pH 7.4 and depolymerizes in an environment of pH 5.5.

### Example 2

This example provides a preparation method for a pH and MPO dual-responsive activated NIR-II CRET nanosystem, which specifically includes the following steps:
(1) 20 mg of luminol, 1 g of sodium bicarbonate and 100 µL of strong aqua ammonia were dissolved in water to obtain a luminol solution at 10 mM;
(2) 1 mg of mercaptopropionic acid-modified AgAuSe QDs were dissolved in water to obtain a solution of QDs at 2 mg/mL;
(3) the luminol solution obtained in step (1) and the quantum dot solution obtained in step (2) were mixed at an average mass ratio of 50:1 and continuously stirred for 1 h at a stirring speed of 300 rpm;
(4) the solution obtained in step (3) was purified three times by ultrafiltration, for 10 minutes each time, to remove excessive luminol to obtain a purified solution containing NIR-II CRET functional units;
(5) 1 mg of fluorenylmethyloxycarbonyl histidine (Fmoc-His) and 2 mmol of GdCl₃ were respectively dissolved in 1 mL of water to obtain an Fmoc-His solution and a GdCl₃ solution, respectively, and 2 mg of chlorin e6 (Ce6) was dissolved in 1 mL of DMSO to obtain a Ce6 solution;
(6) the solution of NIR-II CRET functional units obtained in step (4) and the Fmoc-His solution obtained in step (5) were simultaneously mixed with the GdCl₃ solution and continuously stirred for 30 minutes at a stirring speed of 300 rpm;
(7) all the Ce6 solution obtained in step (5) and all the mixed solution obtained in step (6) were mixed and divided into two portions, which were respectively adjusted to pH = 7.4 and pH = 5.5, and continuously stirred for 1 h, and then concentrated by ultrafiltration to obtain a purified solution of the nanosystem.

As shown in FIG. 2, it shows the morphology characterization images of the NIR-II CRET nanosystem provided in this example. The transmission electron microscopy images show that the size of the nanosystem is about 80 nm at pH 7.4 and about 27 nm at pH 5.5. Furthermore, it can be seen from the figures that the NIR-II CRET nanosystem is successfully prepared at pH 7.4 and depolymerized at pH 5.5.

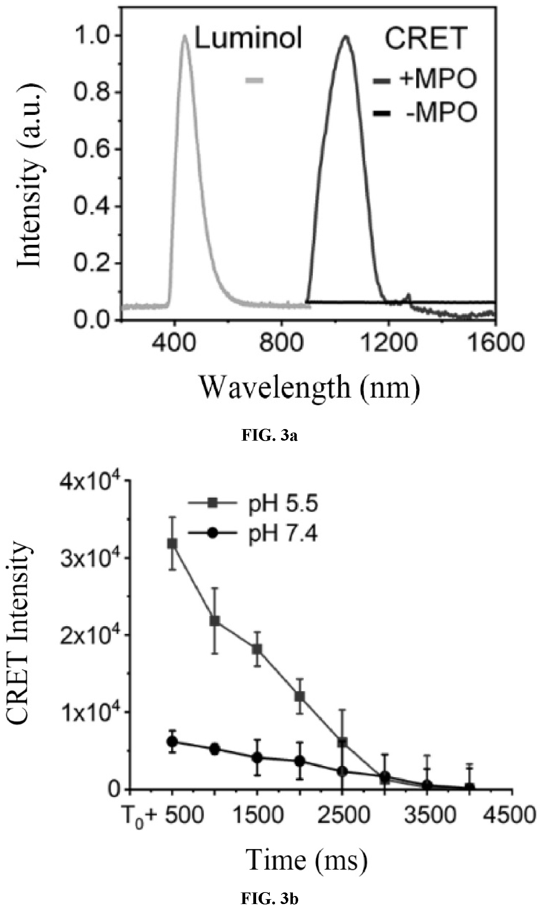

FIG. 3a and 3b present the in vitro CRET spectra and fluorescence signal-time profiles of the NIR-II CRET nanosystem generated via MPO-catalyzed reaction. FIG. 3a confirms that CRET signals are produced through a dual-responsive reaction to both pH and MPO. The quantitative profiles in FIG. 3b further show that the CRET signal exhibit stronger at pH 5.5 than at pH 7.4, indicating a significantly pH-dependent MPO response of the nanosystem.

### Example 3

The NIR-II CRET nanosystem from Example 2 was employed as a diagnostic reagent for the integrated diagnosis and treatment of metastatic SLNs in breast cancer in vivo. The specific steps included the following:
(1) Construction of breast cancer lymphatic metastasis model: the nanosystem was subcutaneously injected into tumor mice and normal mice via mouse paw pads; real-time imaging of the mice were carried out with a Series II 900/1700 In Vivo Imaging System (Suzhou NIR-Optics Co., Ltd., China).; real-time fluorescence imaging was performed to monitor signal changes in the SLNs of both mouse groups; subsequently, the SLN tissues were harvested for histopathological staining analysis..
(2) Postoperative histopathological staining analysis: the lymph node tissues removed above were sectioned and then subjected to immunofluorescence staining and H&E staining. Images of the stained sections were captured by an inverted fluorescence microscope and pathological analysis was conducted. The above *in vivo* fluorescence images and histopathological staining results were compared to analyze the consistency of NIR fluorescence signals, NIR-II CRET signals and histopathological diagnosis of metastatic and normal lymph nodes.

The analytical methods for the above steps are all conventional technical means commonly used in the prior art.

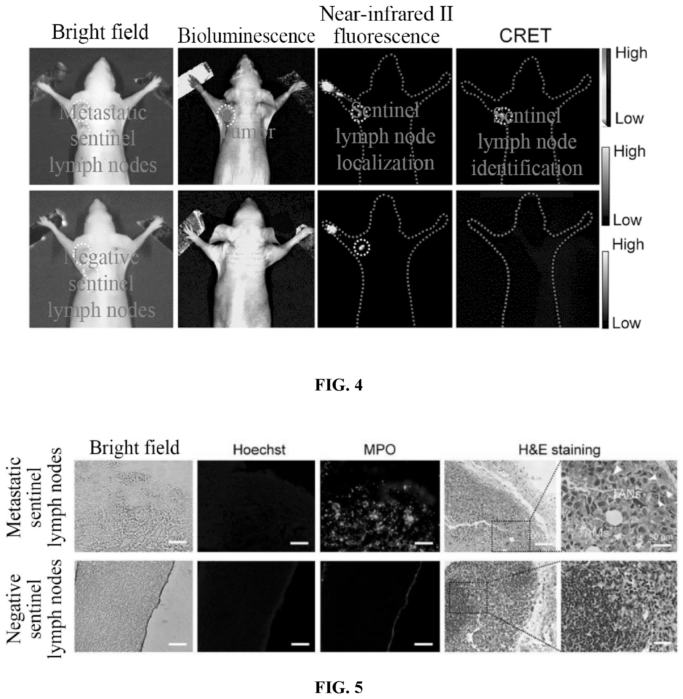

As shown in FIG. 4, accurate localization of breast cancer SLNs was achieved with the nanosystem under 808 nm laser irradiation. Without laser irradiation, metastatic SLNs were specifically identified by NIR-II CRET signals generated by the nanosystem, whereas negative SLNs showed no significant signal. This confirms that detection is based on the pH- and MPO-dual-responsive activation of NIR-II CRET.

As shown in FIG. 5, H&E staining of metastatic SLNs revealed substantial infiltration by TAMs and TANs. Concurrently, immunofluorescence staining showed high MPO expression in these nodes. these aforementioned results suggest that the nanosystem identifies metastatic SLNs through the dual-responsive catalytic generation of a CRET signal.

Therefore, the above results demonstrate that the NIR-II CRET nanosystem of the present disclosure enables integrated diagnosis and treatment of metastatic SLNs in breast cancer. This system exhibits dual responsiveness to pH and MPO: it depolymerizes under acidic conditions and subsequently generates MPO-catalyzed NIR-II CRET signals, thereby allowing for in vivo localization and identification of metastatic SLNs.

The various aspects, embodiments, features and examples of the present disclosure should be regarded as illustrative in all respects and are not intended to limit the present disclosure, the scope of which is defined only by the claims. Without departing from the spirit and scope of the claimed invention, other embodiments, modifications, and applications will be apparent to those skilled in the art.

The use of headings and contents of the specification in the solution of the present disclosure is not meant to limit the present disclosure; each part may be applied to any aspects, embodiments or features of the present disclosure.

Throughout the solution of the present disclosure, where a composition is described as having, comprising, or including particular components or where a process is described as having, comprising, or including particular process steps, it is expected that the composition taught by the present disclosure will also consist essentially of, or consist of, the recited components, and that the process taught by the present disclosure will also consist essentially of, or consist of, a group of the recited process steps.

Unless otherwise specifically stated, the use of the terms "include/includes/including" and "have/has/having" is generally to be understood as open-ended and not restrictive.

It should be understood that the order of the steps or the order in which particular actions are performed is not critical, as long as the teachings of the present disclosure remain operable. In addition, two or more steps or actions can be performed simultaneously.

In addition, the inventor also conducted tests with reference to the foregoing embodiments with other raw materials, process operations, and process conditions described herein, and all obtained relatively ideal results.

Although the present disclosure has been described with reference to illustrative embodiments, it will be understood by those skilled in the art that various other changes, omissions and/or additions may be made and substantial equivalents may be substituted for the elements of the described embodiments without departing from the spirit and scope of the present disclosure. In addition, many modifications may be made to adapt particular situations or materials to the teachings of the present disclosure without departing from the scope of the present disclosure. Therefore, it is not intended herein to limit the present disclosure to the particular embodiments disclosed for carrying out the invention, but rather it is intended that the present disclosure will encompass all embodiments that fall within the scope of the appended claims. Moreover, unless specifically stated, any use of the terms such as first, second, etc. does not indicate any order or importance, but rather the terms such as first, second, etc. are used to distinguish one element from another.

## Claims

1. A near-infrared II chemiluminescence resonance energy transfer (NIR-II CRET) nanosystem, wherein the NIR-II CRET nanosystem is constructed through multicomponent coordinated self-assembly of a pH-responsive amphiphilic short peptide, a metal ion, an NIR-II CRET functional unit, and a photosensitizer;
the NIR-II CRET nanosystem features pH and MPO dual-responsive activation of NIR-II CRET signals.

2. The NIR-II CRET nanosystem according to claim 1, wherein the pH-responsive amphiphilic short peptide is any one of and any combination of two or more of N-fluorenylmethyloxycarbonyl-L-histidine (Fmoc-His), N-fluorenylmethyloxycarbonyl-L-lysine (Fmoc-Lys), N-fluorenylmethyloxycarbonyl-L-arginine (Fmoc-Arg), and N-fluorenylmethyloxycarbonyl-L-cysteine (Fmoc-Cys).

3. The NIR-II CRET nanosystem according to claim 1, wherein
the metal ion is one of or any combination of two or more of gadolinium ion (Gd³⁺), erbium ion (Er³⁺), ferric ion (Fe³⁺), calcium ion (Ca²⁺), copper ion (Cu²⁺), magnesium ion (Mg²⁺), zinc ion (Zn²⁺), and manganese ion (Mn²⁺).

4. The NIR-II CRET nanosystem according to claim 1, wherein the NIR-II CRET functional unit comprises a NIR-II contrast agent and a chemiluminescent compound;
the chemiluminescent compound is luminol or a luminol derivative; and the luminol derivative is selected from 4-aminohexyl-N-ethylisoluminol (ABEI) and/or ethyl luminol (AHEI).

5. The NIR-II CRET nanosystem according to claim 1, wherein the NIR-II contrast agent comprises at least one of the contrast agents including near-infrared quantum dots (NIR QDs).

6. The NIR-II CRET nanosystem according to claim 5, wherein the NIR QDs are modified by one of or any combination of two or more of mercaptopropionic acid, undecanoic acid, glutathione, and lipoic acid.

7. The NIR-II CRET nanosystem according to claim 5, wherein the NIR QDs are one of or any combination of two or more of Ag₂S, Ag₂Se, AgTe, Au:Ag₂Te, and AgAuSe.

8. The NIR-II CRET nanosystem according to claim 1, wherein
the photosensitizer is one of or any combination of two or more of chlorin e6 (Ce6), benzoporphyrin derivative (BPD), 2-(1-hexaoxyethyl)-2-devinyl pyrophosphate (HPPH), and benzoporphyrin monoacid ring A (BPD-MA).

9. A preparation method for the NIR-II CRET nanosystem of any one of claims 1-8, wherein the NIR-II CRET nanosystem is prepared by mixing a pH-responsive amphiphilic short peptide, a metal ion, an NIR-II CRET functional unit and a photosensitizer followed by multicomponent coordinated self-assembly;
the mechanism of multicomponent coordinated self-assembly comprises electrostatic adhesion, metal coordination, hydrophobic interaction, π-π stacking, and other interactions among the pH-responsive amphiphilic short peptide, the metal ion, the NIR-II CRET functional unit and the photosensitizer.

10. The preparation method according to claim 9, wherein the preparation method comprises the following steps:
(1) providing a solution of NIR-II CRET functional units;
an NIR-II contrast agent and a chemiluminescent compound are respectively dissolved in water to obtain a solution of the contrast agent and a solution of the chemiluminescent compound, which are mixed and continuously stirred for 30-120 minutes, and then purified to obtain a solution of the NIR-II CRET functional units;
(2) providing an amphiphilic short peptide solution, a metal ion solution and a photosensitizer solution;
a pH-responsive amphiphilic short peptide is dissolved in water to obtain the amphiphilic short peptide solution;
a metal ion is dissolved in water to obtain the metal ion solution;
a photosensitizer is dissolved in an organic solvent to obtain the photosensitizer solution;
(3) preparing the NIR-II CRET nanosystem;
the purified solution containing NIR-II CRET functional units obtained from step (1) is mixed with the amphiphilic short peptide solution and the metal ion solution from step (2), followed by continuously stirring for 10-40 minutes; the photosensitizer solution prepared in step (2) is then added and mixed uniformly, adjusted to neutral pH and purified, and concentrated to obtain the NIR-II CRET nanosystem.

11. The preparation method according to claim 10, wherein
in step (1) and step (4), the purification is purification by ultrafiltration;
the preparation method for the chemiluminescent compound solution comprises dissolving the chemiluminescent compound in an alkaline solution; and the pH of the alkaline solution is about pH 8.0-9.0;
a mass ratio of the NIR-II contrast agent to the chemiluminescent compound is in a range of 1-10:0.1-1;
in step (2), the organic solvent is dimethyl sulfoxide;
the concentration of the amphiphilic short peptide solution is 1-5 mg/mL;
the concentration of the metal ion solution is 5-20 mmol/mL;
the concentration of the photosensitizer solution is 1-10 mg/mL;
in step (3), a mass ratio of the NIR-II contrast agent, the chemiluminescent compound, the amphiphilic short peptide, the metal ion and the photosensitizer contained in the NIR-II CRET nanosystem is in a range of 1-10:50-100:5-50:50-200:1-5.

12. The preparation method according to claim 10, wherein the average size of the NIR-II CRET nanosystem under neutral conditions is in a range of 20-500 nm.

13. Application of the NIR-II CRET nanosystem of any one of claims 1-8 in the preparation of a diagnostic reagent for SLNs in breast cancer.

14. A diagnostic reagent for sentinel lymph nodes (SLNs) in breast cancer, at least comprising the NIR-II CRET nanosystem of any one of claims 1-8, the diagnostic reagent shows dual responsiveness to pH and MPO, and produces NIR-II CRET signals catalyzed by MPO after depolymerization reaction under acidic conditions, which enables the integrated localization and identification for the diagnosis and treatment of metastatic SLNs in breast cancer at an *in vivo* level.
